(19) Europäisches Patentamt
European Patent Office
Office européen des brevets

(11) Veröffentlichungsnummer: **0 040 353**
**B1**

(12) # EUROPÄISCHE PATENTSCHRIFT

(45) Veröffentlichungstag der Patentschrift: **14.12.83**

(51) Int. Cl.³: **C 07 D 493/18,** C 07 D 311/94 // (C07D493/18, 319/00, 311/00)

(21) Anmeldenummer: **81103409.9**

(22) Anmeldetag: **06.05.81**

(54) **Verfahren zur Herstellung von Lineatin.**

(30) Priorität: **16.05.80 DE 3018635**

(43) Veröffentlichungstag der Anmeldung: **25.11.81 Patentblatt 81/47**

(45) Bekanntmachung des Hinweises auf die Patenterteilung: **14.12.83 Patentblatt 83/50**

(84) Benannte Vertragsstaaten: **AT CH DE FR GB LI SE**

(56) Entgegenhaltungen:
**DE - A - 1 961 433**
**DE - A - 2 730 988**
**DE - B - 1 593 387**
**DE - B - 2 129 507**

(73) Patentinhaber: **CELAMERCK GmbH & Co. KG, Binger Strasse 173, D-6507 Ingelheim am Rhein (DE)**

(72) Erfinder: **Slessor, Keith N., Department of Chemistry Simon Fraser University, Burnaby British Columbia, V5A 1S6 (CA)**
Erfinder: **Oehlschlager, Allan C., Department of Chemistry Simon Fraser University, Burnaby British Columbia, V5A 1S6 (CA)**
Erfinder: **Johnston, Blair D., Department of Chemistry Simon Fraser University, Burnaby British Columbia, V5A 1S6 (CA)**
Erfinder: **Pierce, Harold, D., Jr., Department of Chemistry Simon Fraser University, Burnaby British Columbia, V5A 1S6 (CA)**
Erfinder: **Grewal, Sukhjit K., Department of Chemistry Simon Fraser University, Burnaby British Columbia, V5A 1S6 (CA)**
Erfinder: **Wickremesinghe, Kirthi G. L., Department of Chemistry Simon Fraser University, Burnaby British Columbia, V5A 1S6 (CA)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

Verfahren zur Herstellung von Lineatin

Die Erfindung betrifft ein Verfahren zur Herstellung racemischen Lineatins (3,3,7-Trimethyl-2,9-dioxatricyclo[3.3.1.0]nonan) einem Aggregationspheromon des gestreiften Nutzholzborkenkäfers *(Trypodendron lineatum)*, Formel

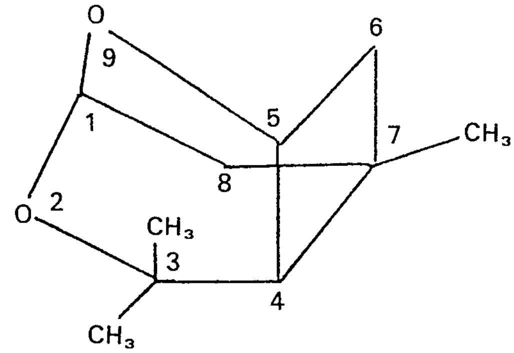

(I)

Lineatin eignet sich zum Anlocken des genannten Käfers und kann daher verwendet werden, um seine Bekämpfung zu erleichtern.

Das erfindungsgemässe Verfahren ist dadurch gekennzeichnet, dass man gemäss dem Reaktionsschema

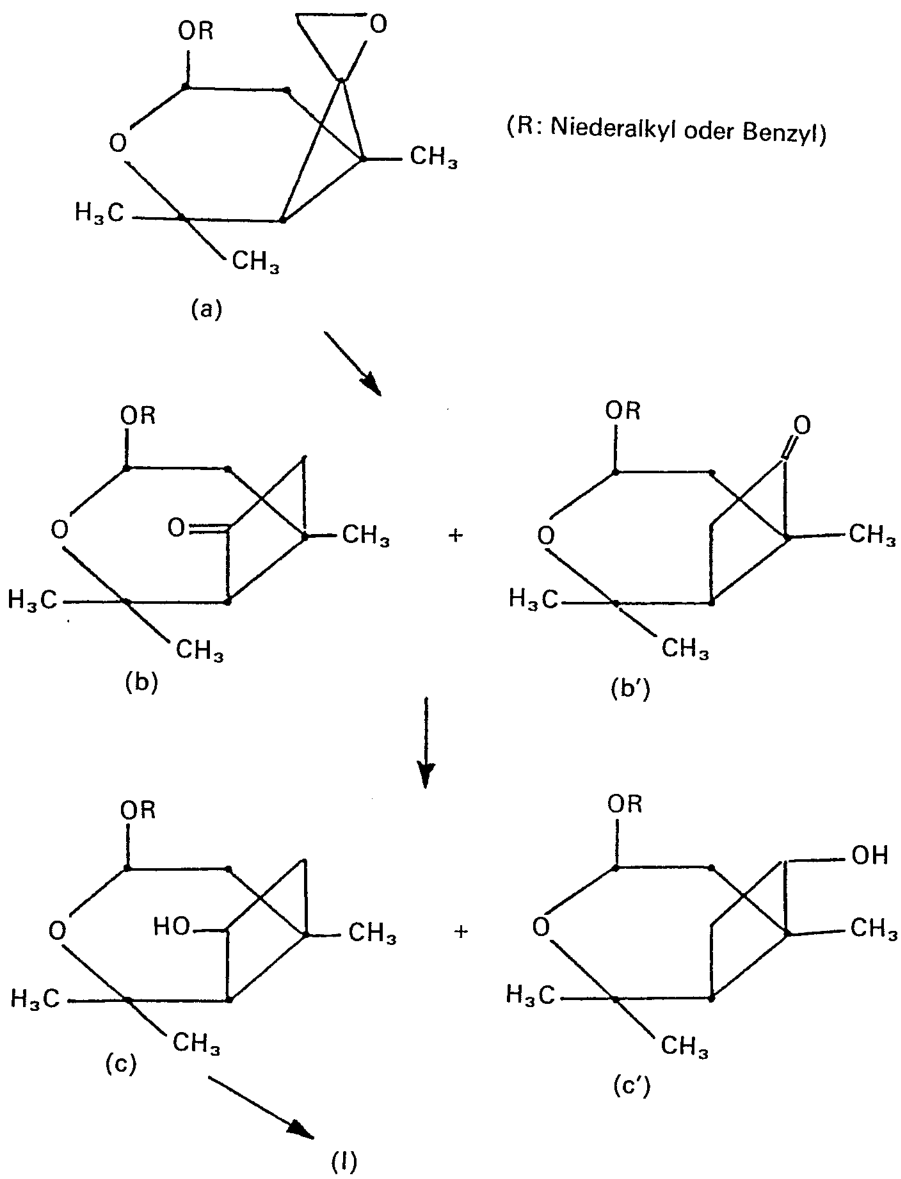

(R: Niederalkyl oder Benzyl)

(a)

(b) + (b')

(c) + (c')

(I)

1. die Spiroverbindung a zu dem Gemisch der Cyclobutanone b und b' umlagert,

2. das Cyclobutanon b oder das Gemisch der Cyclobutanone zu den entsprechenden Alkoholen c bzw. dem Gemisch c/c' reduziert, und schliesslich

3. den Alkohol c in das Lineatin I überführt.

Die Umlagerung 1 gelingt durch Einwirkung von Alkalihalogeniden, z.B. Lithiumbromid, in einem inerten Lösungsmittel, z.B. Acetonitril, unter neutralen Bedingungen, vorzugsweise in der Wärme. Zusatz von Lithiumcarbonat ist vorteilhaft. Die Verbindungen b und b' bilden sich etwa im Verhältnis 4:1 in hoher Ausbeute.

Sie können vor der Weiterverarbeitung getrennt werden, etwa durch Chromatographie. Jedoch ist es günstiger, die Trennung erst auf der Stufe der Alkohole c/c' vorzunehmen.

Zur Reduktion von b/b' gemäss 2 dienen bevorzugt komplexe Hydride, insbesondere Alkaliborhydride bzw. Derivate von diesen; sie werden in üblicher Weise angewendet. Die erhaltenen Alkohole c und c' werden chromatographisch getrennt.

Gemäss 3 wird der Alkohol c in racemisches Lineatin übergeführt. Dies gelingt durch Säurekatalyse, z.B. mit Carbonsäuren, etwa Essigsäure, Schwefelsäure, Halogenwasserstoffsäuren und besonders p-Toluolsulfonsäure.

Der Ausgangsstoff a kann auf folgendem Weg erhalten werden:

I

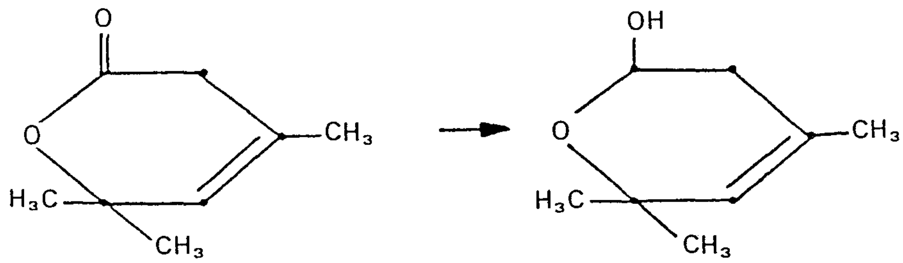

(d) (e)

II

(e)

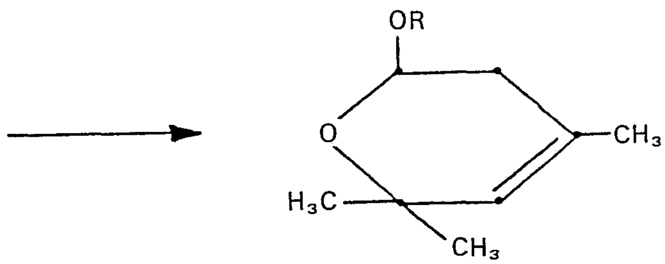

(f)

(R: Niederalkyl oder Benzyl)

III

(f) →

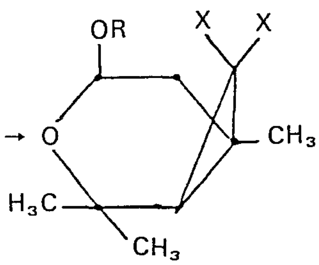

\+

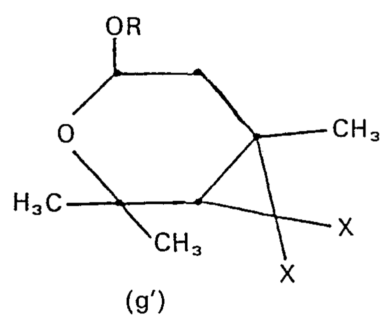

(g) (g')

(X: gleich Halogen)

IV

(g) + (g')

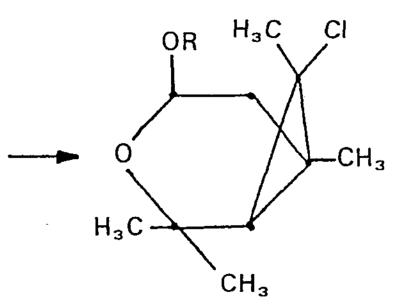

\+

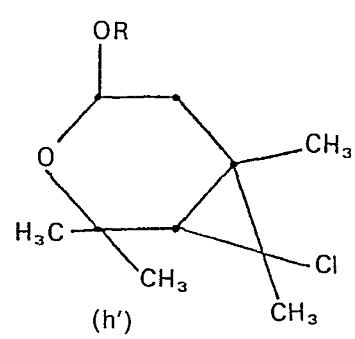

(h) (h')

V (h) + (h')

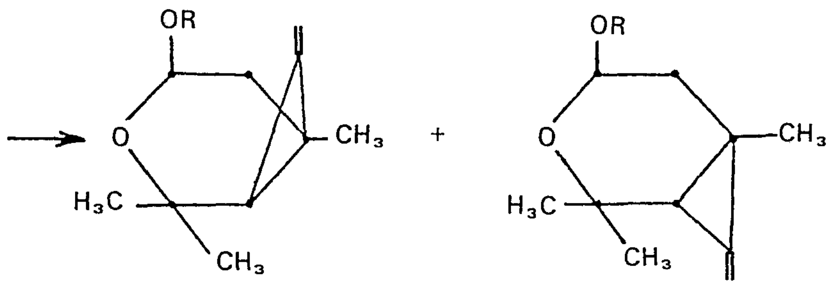

\+

(i) (i')

VI (i) + (i')

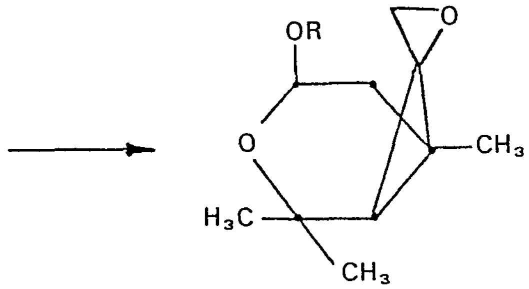

(a)

Ausgehend von dem literaturbekannten cyclischen Lacton der 5-Hydroxy-3,5-dimethyl-3-hexencarbonsäure d wird zunächst durch Reduktion, bevorzugt mit komplexen Hydriden, das Halbacetal e hergestellt, das durch Alkylierung bzw. Aralkylierung in das Acetal f übergeführt werden kann; bevorzugt ist R gleich Niederalkyl. In Stufe III lagert man an das Acetal ein Dihalogencarben, vorzugsweise Dichlorcarben, an. Es entstehen die isomeren Dihalocyclopropane g und g' im Verhältnis von etwa 2:1. Die Annelierung des Dihalogencyclopropanringes wird vorzugsweise unter Phasentransferkatalyse vorgenommen. Nach der Methode von Kitatani („Bull. Chem. Soc. Jap.", *50,* 3288 (1977)] lässt sich dieses Gemisch metallieren und anschliessend methylieren. Für die Ausbeute an gewünschtem Endomethylierungsprodukt h und h' ist eine tiefe Reaktionstemperatur günstig.

Als Metallierungsreagenz kann jede metallorganische Base dienen, vorzugsweise Butyllithium. Um Endoalkylderivate zu erhalten, müssen die Chlor- und Lithiumisomeren Gelegenheit haben zu äquilibrieren.

Als günstig wird dafür eine Zeitspanne von 10 bis 60, vorzugsweise etwa 30 min, angesehen. In der V. Stufe wird die Chlormethyl/Cyclopropan-Isomerenmischung h/h' mit einer Base geeigneter Stärke, vorzugsweise Kalium-tert.-butylat in Dimethylsulfoxid versetzt. Es entstehen die entsprechenden Methylencyclopropane i und i' im Verhältnis 3:1. Unterwirft man diese Mischung der Reaktion nach Prileschajew („Reaktionen der organ. Chemie", S. 560; Hüthig Verlag, Heidelberg [1976]), so bildet sich vorwiegend das Oxiran a, das als Ausgangsstoff für die erfindungsgemässe Umsetzung dient.

Im folgenden werden die Herstellung des Ausgangsstoffes und ein Beispiel für die experimentelle Durchführung der erfindungsgemässen Umsetzung näher beschrieben.

Herstellung des Ausgangsprodukts

a) *2,2,4-Trimethyl-6-methoxy-5,6-dihydro-[2H]-pyran*

Eine Lösung von 157 g (1,1 mol) 5-Hydroxy-3,5-dimethyl-3-hexencarbonsäurelacton in einer Mischung von 200 ml trockenem Tetrahydrofuran und 60 ml Äthyläther wird auf −20 bis −30° C gekühlt. Im Lauf von 15 min wird zu dieser Lösung eine Aufschlämmung von 11,2 g (0,29 mol) Lithiumaluminiumhydrid in 320 ml trockenem Tetrahydrofuran gegeben. Man lässt dann die Mischung sich auf Raumtemperatur erwärmen und giesst sie nach 30 min auf eine Mischung von 800 g Eis und 33 ml konz. Schwefelsäure. Die ätherische Schicht wird abgetrennt und die wässerige Phase zweimal mit je 200 ml Äther extrahiert. Die vereinigten ätherischen Extrakte werden mit 100 ml 5%iger Natriumhydrogencarbonatlösung und mit 100 ml gesättigter wässeriger Natriumchloridlösung gewaschen, dann mit Natriumsulfat getrocknet und im Vakuum eingeengt.

Die konzentrierte Reaktionsmischung wird ohne Reinigung mit 100 ml Orthoameisensäuretrimethylester und 4 g Ammoniumnitrat in 40 ml Methanol 12 h bei Raumtemperatur gerührt und dann 4 h unter Rückfluss gekocht. Nach dieser Zeit giesst man die Mischung in 300 ml Wasser und extrahiert dreimal mit je 200 ml Chloroform. Der Extrakt wird mit 100 ml gesättigter wässeriger Natriumchloridlösung gewaschen, mit Magnesiumsulfat getrocknet und im Vakuum eingeengt. Das Konzentrat wird dann unter vermindertem Druck destilliert, wobei 102 g (58% d.Th.) der Titelverbindung erhalten werden. Siedepunkt: 62-67° C bei 20 mbar.

b) *Syn- und Anti-2,2,6-trimethyl-4-methoxy-7,7-dichlor-3-oxabicyclo[4.1.0]heptan*

Zu einer Lösung von 100 g (0,64 mol) der nach a erhaltenen Verbindung in 250 ml Chloroform

werden 2 g (5,5 mmol) Cetyltrimethylammoniumbromid und eine gekühlte Lösung von 250 g Natriumhydroxid in 250 ml Wasser zugeführt. Die Zweiphasenreaktion erfolgte unter kräftigem Rühren. Nach 3 h mässigt sich die anfänglich stark exotherme Reaktion und die Analyse zeigt, dass die Umsetzung zu etwa 50% erfolgt ist. Nach weiterem 16stündigem Rühren ist die Umsetzung zu über 95% abgelaufen. Die Reaktionslösung wird mit 1 l, Wasser verdünnt, die Chloroformphase abgetrennt und die wässerige Phase zweimal mit je 300 ml Methylenchlorid extrahiert. Die vereinigten Extrakte werden zweimal mit 500 ml Wasser unter Zusatz einer geringen Menge Magnesiumsulfat gewaschen. Die organische Phase wird getrocknet und im Vakuum eingeengt. Nach fraktionierter Destillation über Natriumhydroxidperlen erhält man die Titelverbindungen im Verhältnis 2, 1:1; Ausbeute: 145 g (94% d.Th.); Siedepunkt: 70-80° C (0,133 mbar).

c) *2,2,6,7-Tetramethyl-4-methoxy-7-chlor-3-oxabicyclo[4.1.0]heptan*

Eine Lösung von 24 g (100 mmol) des nach b erhaltenen Gemischs in 300 ml trockenem Tetrahydrofuran wird auf −100° C gekühlt. Unter Stickstoff lässt man bei dieser Temperatur im Laufe von 30 min eine Mischung von 90 ml Butyllithium, 2,5M in Hexan, entspricht 225 mmol, zutropfen. Nach weiteren 30 min gibt man zu dem Reaktionsgemisch 30,4 g (210 mmol) Methyljolid im Laufe von 20 min. Die Temperatur wird dabei im Bereich von −100 bis −80° C gehalten. Im Laufe von 10-12 h lässt man dann die Temperatur des Reaktionsgemisches auf Raumtemperatur ansteigen. Die Lösung wird sodann mit 1 l Wasser verdünnt und dreimal mit 200 ml Petroläther (Siedetemperatur 30-60° C) extrahiert. Die organische Phase wird mit Natriumsulfat getrocknet und im Vakuum eingeengt. Bei der fraktionierten Destillation werden 17,5 g einer Fraktion mit dem Siedebereich 55-60° C (1,0 mbar) erhalten.

Sie besteht ganz überwiegend aus dem Gemisch der Titelverbindungen.

d) *Syn- und Anti-2,2,6-trimethyl-4-methoxy-7-methylen-3-oxabicyclo[4.1.0]heptan*

Zu einer Suspension von 20 g (178 mmol) Kalium-tert.-butylat in 60 ml trockenem Dimethylsulfoxid unter Stickstoff werden bei 90° C 12 g (55 mmol) des nach c erhaltenen Isomerengemischs im Laufe von 10 min zugegeben. Nach 15 min wird das Reaktionsgemisch gekühlt, mit 500 ml Wasser verdünnt und 24 h kontinuierlich mit Pentan extrahiert. Nach Entfernung des Pentans wird der Rückstand im Vakuum destilliert. Es werden 5,63 g (56% d.Th.) eines 3: 1-Gemischs der Titelverbindungen erhalten; Siedepunkt 60-65° C (9 mbar).

e) *2,2,6-Trimethyl-4-methoxy-7,8-epoxy-3-oxabicyclo[4.1.0]heptan*

Zu 12,2 g (61,6 mmol) der nach d erhaltenen Isomeren in 300 ml Methylenchlorid gibt man 1 l eines Phosphatpuffers (pH 6,4, 35,5 g $Na_2HPO_4$ und 34 g $KH_2PO_4$ in 1 l Wasser).

Im Laufe von 10 min werden hierzu unter Rühren 21 g p-Nitroperbenzoesäure (98% Reinheit; 112 mmol) gegeben. Das Reaktionsgemisch wird 14 h bei Raumtemperatur gerührt, wonach das Oxidationsmittel vollständig aufgebraucht ist. Man trennt dann die Phasen und wäscht die wässerige Phase mit 100 ml Methylenchlorid. Die vereinigten organischen Lösungen werden mit Magnesiumsulfat getrocknet, im Vakuum eingeengt und einer Vakuumdestillation unterworfen. Man erhält 8,1 g (66% d.Th.) der Titelverbindung; Siedepunkt 55-60° C (0,5 mbar).

*Beispiel*

a) *2,2,6-Trimethyl-4-methoxy-3-oxabicyclo-[4.2.0]octan-8-on und 7-on*

Zu 5,8 g (29 mmol) des gemäss e erhaltenen Epoxids in 75 ml Acetonitril werden 250 mg Lithiumcarbonat und 10,2 g (117 mmol) Lithiumbromid (12 h bei 125° C getrocknet und sofort verwendet) gegeben. Die Lösung wird gerührt, um das Lithiumbromid zu lösen und 30 min auf 60° C erwärmt. Das Reaktionsgemisch wurde auf Raumtemperatur abgekühlt, mit 100 ml Wasser verdünnt und viermal mit je 100 ml Pentan extrahiert. Die vereinigten Extrakte werden mit Magnesiumsulfat getrocknet und unter Verwendung einer Vigreaux-Kolonne eingeengt. Man destilliert den Rückstand nach Zugabe von 10 mg Lithiumcarbonat und erhält 4,8 g (83% d.Th.) eines Produkts; Siedepunkt 55-60° C bei 0,4 mbar.

Das Destillat enthält die Titelverbindungen im Verhältnis 4:1.

b) *2,2,6-Trimethyl-4-methoxy-3-oxabicyclo-[4.2.0]octan-8-ol und 7-ol*

Zu 3,2 g (16 mmol) der 4:1-Mischung der nach a erhaltenen bicyclischen Ketone in 30 ml 95%igen Äthanols werden 0,611 g (16 mmol) Natriumborhydrid gegeben. Das Gemisch wird über Nacht bei Raumtemperatur gerührt und dann mit 150 ml Wasser verdünnt. Man extrahiert mit 5 Portionen von je 50 ml Äthyläther. Die vereinigten Extrakte werden mit Natriumsulfat getrocknet und im Vakuum eingeengt. Man erhält 3,2 g eines Öls. Das Rohprodukt wird an 200 g Silicagel chromatograpahiert (Hexan/Äthylacetat 1:1), Verfahren nach Still („J. Org. Chem.", *43*, 2923 [1978]). Es werden Fraktionen von 25 ml gesammelt. Die Fraktionen 20 bis 22 enthalten neben Verunreinigungen den gewünschten Alkohol mit der 8-OH-Gruppe ($R_F$=0,55, 0,468 g). Der Hauptanteil dieses Alkohols findet sich in den Fraktionen 23 bis 29 (1,3 g, 40% d.Th.). Auch die Fraktionen 30 bis 33 enthalten diesen Alkohol, neben dem Isomerenalkohol mit der 7-OH-Gruppe ($R_F$ =0,40). Der 8-Alkohol wird durch das NMR-Spektrum charakterisiert:

$1_H$-NMR: δ 4,98 (1H, $C_4$, t, J=7 Hz), 4,42 (1H, $C_8$, m), 3,42 (3H, $OCH_3$, s), 2,7-1,5 (6H, OH, $C_1$, $C_5$, $C_7$, m), 1,41 (3H, $CH_3$, s), 1,24 (3H, $CH_3$, s), 1,16 (3H, $CH_3$, s).

c) *(±)-3,3,7-Trimethyl-2,9-dioxatricyclo-[3.3.1.0]nonan*

Zu 1,3 g (6 mmol) 2,2,6-Trimethyl-4-methoxy-3-oxabicyclo[4.2.0]octan-8-ol in einer Mischung aus 20 ml Pentan und 0,5 ml Methanol werden einige Kristalle p-Toluolsulfonsäure (ca. 1 mg) gegeben. Das Gemisch wird 1 h bei Raumtemperatur gerührt, durch neutrales Aluminiumoxid filtriert und im Vakuum eingeengt. Die Vakuumdestillation (unter Zugabe von 2 mfg Lithiumcarbonat) ergibt 0,71 g Lineatin (65% d.Th.); Siedepunkt 70°C (16 mbar). Nach der gaschromatographischen Analyse hat das Produkt einen Reinheitsgrad von 98%. Es wird durch NMR charakterisiert.

$1_H$-NMR: δ 4,91 (1H, $C_1$, b, s), 4,39 (1H, $C_5$, m), 2,2-1,5 (5H, $C_4$, $C_6$, $C_8$, m), 1,15 (6H, $2CH_3$, s), 1,11 (3H,$CH_3$, s).

**Patentansprüche**

1. Verfahren zur Herstellung von Lineatin, dadurch gekennzeichnet, dass man 2,2,6-Trimethyl-4-R-oxy-7,8-epoxy-3-oxabicyclo[4.1.0]heptan, Formel

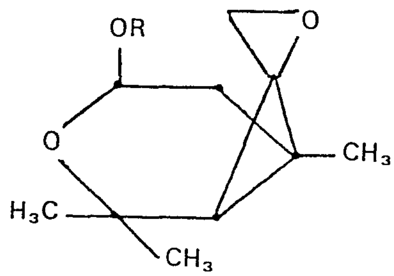
(a)

wobei R Niederalkyl oder Benzyl bedeutet, unter der Einwirkung von Alkalihalogeniden in einem inerten Lösungsmittel unter neutralen Bedingungen in der Wärme zu einem Gemisch von 2,2,6-Trimethyl-4-R-oxy-3-oxybicyclo[4.2.0]octan-8-on bzw. -7-on, Formeln

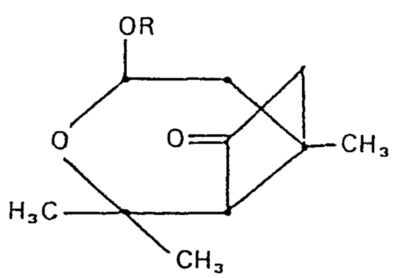
(b)

und 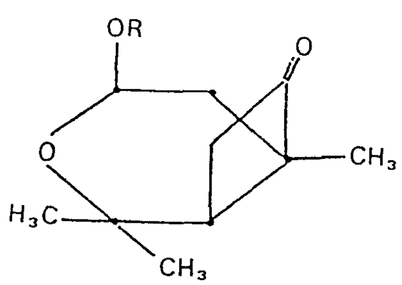 (b')

umlagert, dass man anschliessend die Mischung der Ketone mit einem komplexen Hydrid unter üblichen Bedingungen zu den entsprechenden Alkoholen, Formeln

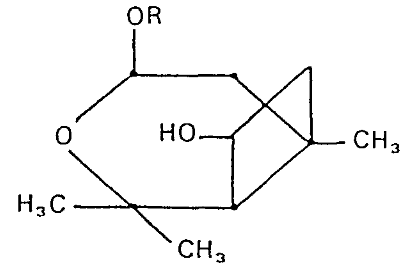
(c)

und 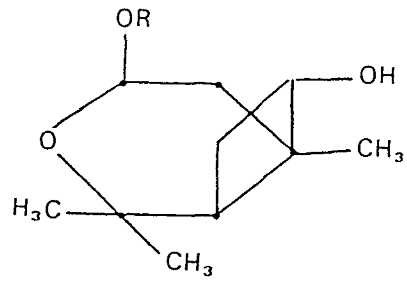 (c')

reduziert, und dass man nach Abtrennung des Alkohols c diesen durch Säurekatalyse in racemisches Lineatin überführt.

2. Verfahren nach Anspruch 1, dadurch gekennzeichnet, dass man für die Umlagerung der Verbindung a Lithiumbromid anwendet.

3. Vefahren nach Anspruch 2, dadurch gekennzeichnet, dass als Lösungsmittel Acetonitril verwendet wird.

4. Verfahren nach Anspruch 1, dadurch gekennzeichnet, dass als Mittel zur Reduktion der Verbindungen b und b' Natriumborhydrid verwendet wird.

5. Verfahren nach Anspruch 1, dadurch gekennzeichnet, dass zur Überführung des Alkohols c in Lineatin p-Toluolsulfonsäure als Katalysator verwendet wird.

6. 2,2,6-Trimethyl-4-R-oxy-3-oxybicyclo-[4.2.0]octan-8-on, worin R die in Anspruch 1 gegebene Bedeutung hat.

**Claims**

1. Process for the preparation of lineatin, characterized in that 2,2,6-trimethyl-4-R-oxy-7,8-epoxy-3-oxabicyclo[4.1.0]heptane, of formula:

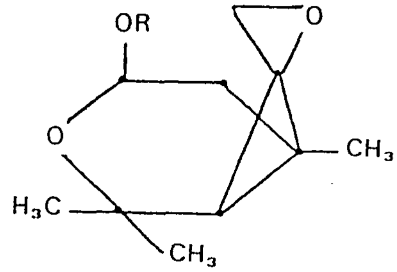
(a)

wherein R represents lower alkyl or benzyl, is rearranged, under the effect of alkali halides, in an inert solvent under neutral conditions and in the warm, to form a mixture of 2,2,6-trimethyl-4-R-oxy-3-oxy-bicyclo[4.2.0]octan-8-one and -7-one of formulae:

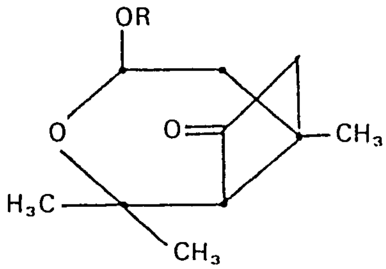 (b)

and 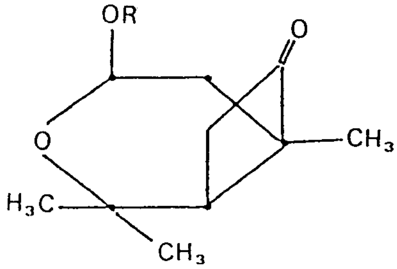 (b')

and subsequently the mixture of ketones is reduced with a complex hydride under conventional conditions to form the corresponding alcohols of formulae:

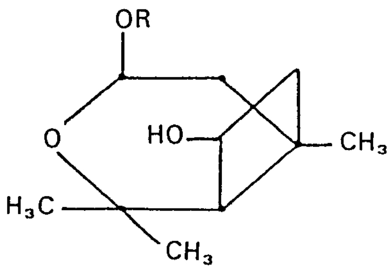 (c)

and 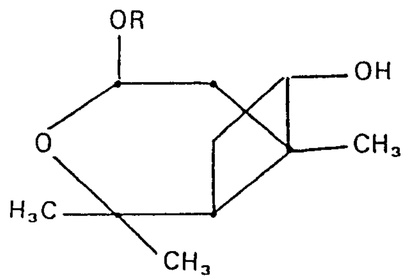 (c')

and after separation of the alcohol (c) the latter is converted into racemic lineatin by acid catalysis.

2. Process as claimed in claim 1, characterized in that lithium bromide is used for the rearrangement of compound (a).

3. Process as claimed in claim 2, characterized in that acetonitrile is used as the solvent.

4. Process as claimed in claim 1, characterized in that sodium borohydride is used as the agent for reducing the compounds (b) and (b').

5. Process as claimed in claim 1, characterized in that p-toluenesulphonic acid is used as the catalyst for converting the alcohol (c) into lineatin.

6. 2,2,6-Trimethyl-4-R-oxy-3-oxabicyclo-[4,2,0]octan-8-one wherein R has the meaning given in claim 1.

**Revendications**

1. Procédé pour la préparation de la linéatine, caractérisé en ce qu'on transpose le 2,2,6-triméthyl-4-R-oxy-7,8-époxy-3-oxabicyclo[4.1.0] heptane de formule:

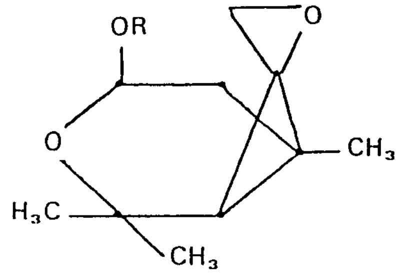 (a)

dans laquelle R signifie alcoyle inférieur ou benzyle, sous l'action d'halogénures alcalins dans un solvant inerte, dans des conditions neutres, à chaud, en un mélange de 2,2,6-triméthyl-4-R-oxy-3-oxy-bicyclo[4.2.0]octane-8-one, ou respectivement 7-one, de formules:

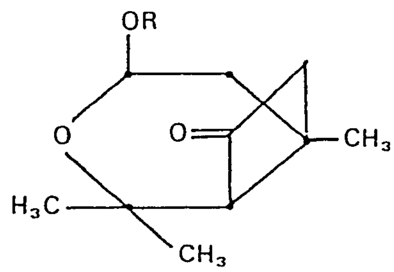 (b)

et 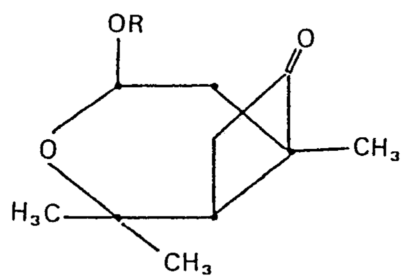 (b')

en ce qu'on réduit ensuite le mélange des cétones au moyen d'un hydrure complexe, dans des conditions habituelles, en les alcools correspondants de formules:

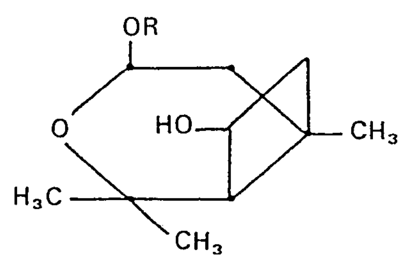 (c)

et 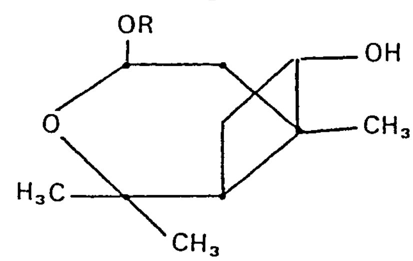 (c')

et en ce que, après séparation de l'alcool (c), on

transforme celui-ci en linéatine racémique par catalyse acide.

2. Procédé selon la revendication 1, caractérisé en ce que, pour la transposition du composé (a), on utilise du bromure de lithium.

3. Procédé selon la revendication 2, caractérisé en ce qu'on utilise de l'acétonitrile comme solvant.

4. Procédé selon la revendication 1, caractérisé en ce qu'on utilise du borohydrure de sodium comme agent pour la réduction des composés (b) et (b').

5. Procédé selon la revendication 1, caractérisé en ce que, pour la transformation de l'alcool (c), en linéatine, on utilise comme catalyseur de l'acide p-toluènesulfonique.

6. 2,2,6-Triméthyl-4-R-oxy-3-oxybicyclo-[4.2.0]octane-8-one dans laquelle R a la signification donnée dans la revendication 1.